# EUROPEAN PATENT SPECIFICATION

(11) **EP 2 901 993 B1**
(45) Date of publication and mention of the grant of the patent: **14.09.2016**
(21) Application number: 14153461.0
(22) Date of filing: 31.01.2014
(51) Int. Cl.: A61F 13/537, A61F 13/15, A61F 13/539

(54) **Undulated structure for an absorbent article**
Gewellte Struktur für einen saugfähigen Artikel
Structure ondulée pour article absorbant

(43) Date of publication of application: 05.08.2015
(73) Proprietor: Ontex BVBA, 9255 Buggenhout (BE)
(72) Inventor: Weber, Ainas, 56727 Mayen (DE); DE POORTER, Annick, 9320 Erembodegem-Aalst (BE)
(74) Representative: Brantsandpatents bvba

(56) References cited:
- EP-A1- 0 788 874
- EP-A1- 1 873 292
- US-A- 4 578 070
- US-A- 5 865 824
- US-A1- 2001 027 305
- US-A1- 2003 073 967
- US-A1- 2006 122 572
- US-A1- 2010 201 020

## Description

### TECHNICAL FIELD

The present invention is of particular importance to the field of hygiene products, in particular diapers.

The present invention relates to an acquisition and distribution layer in an absorbent article, to an absorbent article such as a diaper made of such layered structure, to methods for manufacturing said layered structure and said absorbent article, as well as to the use of a layered structure according to the invention in an absorbent article, particularly a diaper.

### BACKGROUND

A variety of absorbent articles that are adapted to absorb body fluids are well known. Examples of absorbent articles include diapers, incontinent articles, and sanitary napkins.

One problem associated with known absorbent articles is waste product leakage, which may contaminate clothing articles that contact the absorbent article, such as pants, shirts, and bedding. The amount of leakage experienced by a wearer can be reduced by increasing the rate at which the liquid enters the absorbent core. Therefore, an absorbent article wherein liquid rapidly penetrates the topsheet and is contained in the absorbent core will experience less leakage than an absorbent article wherein liquid is able to run across the topsheet before penetrating into the absorbent core. Reducing run-off, therefore, reduces the amount of leakage experienced with an absorbent article.

Another problem associated with absorbent articles is dryness of the skin contacting surface of the article. Generally, the drier the skin contacting surface, the more comfortable the absorbent article. Attempts have been made to reduce surface wetness in disposable diaper structures. For example, U.S. Pat. No. 3,945,386 issued to Anczurowski on Mar.23, 1976 and U.S. Pat. Nos. 3,965,906 and 3,994,299 issued to Karami on Jun. 29, 1976 and Nov. 30, 1976, respectively, teach diaper structures having a perforated thermoplastic film interposed between the topsheet and the absorbent core. U.S. Pat. No. 4,324,247 issued to Aziz on Apr. 13, 1982 describes an effort directed to both reducing run-off and reducing the surface wetness of absorbent articles.

In addition to the dryness of the skin contacting surface, the feel of the skin contacting surface is also an important consideration. One problem is that some consumers do not like the plastic feel associated with formed films. A number of efforts have been directed at improving the feel of the surface of absorbent articles. One example is described in U.S. Pat. No. 3,967,623 issued to Butterworth, et al. The Butterworth patent teaches an absorbent pad having a facing sheet made of a perforated thermoplastic web that has an integral fibrous or sueded outer surface. The products described in most of the above references, however, are less than ideal in achieving a good combination of all three desired properties of reduced surface run-off, improved ability to prevent a feeling of wetness of the topsheet, and improved feel.

US2002062113A1 discloses an absorbent article having a topsheet and an absorbent core material. The acquisition distribution layer is located between the topsheet and the absorbent core material. The acquisition distribution layer is made of a three dimensional apertured film that defines a large void volume space between the acquisition distribution layer and the absorbent core material. The acquisition distribution layer provides high void volume for lateral spillage during repeated insult moments because the top sheet, which is in contact with the user, is held away from dispersing fluid that is unabsorbed by saturated core material. The void volume space provides a pathway for unabsorbed fluid to flow over the top plane of saturated core regions to unsaturated regions of the core material for absorption. The void volume space allows this migration of fluid to occur without the fluid coming into contact with the topsheet, thereby avoiding a feeling of wetness for a wearer. A problem with such an ADL is that fluid from an insult largely remains to a large extent at or at least near the position of the insult.

A transfer layer, which is also known in the art as an acquisition and distribution layer or "ADL", has been used in absorbent articles. Both nonwoven webs and three-dimensional formed films have found use as transfer layer in the past. A transfer layer is typically positioned between the topsheet and the absorbent core and generally improves the efficiency of the article to absorb and retain fluids. For example, transfer layers have been used to provide void volume, which serves as a temporary reservoir to collect and hold fluids until the fluids can be absorbed by the core. In addition, as mentioned in U.S. Patent 4,324,247, transfer layers have been employed to promote lateral flow of fluids in a direction generally parallel to the plane of the transfer layer, thereby permitting more of the core to be used to absorb fluids. See, for example U.S. Patent 4,324,247.

EP2353562A1 discloses a composite particularly suited for use as a transfer layer in an absorbent article, which has a first layer comprising a formed film having a plurality of capillary-sized apertured protuberances and a plurality of two-dimensional drains, and a second layer in intimate contact with an apertured end of said capillary-sized protuberances and spaced from the first layer in a z-direction, the second layer is either a three-dimensional apertured formed film or a nonwoven web. In embodiments where the second layer is a three-dimensional formed film, the film contains a plurality of capillary sized protuberances that are of smaller diameter compared to the protuberances in the first layer and optionally also contains a plurality of drains, which can be either two-dimensional or three-dimensional, or combinations thereof. Also here, a problem with such an ADL is that fluid from an insult largely remains to a large extent at or at least near the position of the insult.

Document EP 1 873 292 A1 discloses a non-woven fabric, characterized in that it is multilayer, with variable density and with longitudinal channels, and is constituted by a non-bonded ply, having non-absorbent hydrophilic characteristics, rested on a bonded ply, having absorbent characteristics, bonded together along parallel lines. A method for manufacturing the non-woven fabric, in which a non-bonded ply is deposited on a bonded ply and is connected to the bonded ply with a particular spunlacing system, which allows the binding of the two plies only along binding lines, forming troughs in the non-bonded ply, while longitudinal ridges remain in the gaps between the binding lines, the fibers of the non-bonded ply remaining soft and loose in the gaps, providing the composite product.

Document US 5 865 824 A discloses an absorbent structure in which an initially flat, dense structure becomes a three-dimensional, high bulk, channeled structure upon wetting. The structure offers directionality in fluid transport to improve the distribution of fluid in longitudinal articles. The self-bulking of the wetted article can also lead to improved fit in articles such as diapers and in general increases the void volume of the wetted article for high absorbent capacity.

Document US 2006/122572 A1 discloses a transfer layer of liquid fluids and an absorbent article incorporating the transfer layer, like a diaper, a sanitary napkin or a similar product, which has a permeable cover below which there is disposed a nonwoven transfer layer for liquid fluids followed beneath by an absorbent core which retains the fluids. In the transfer layer there is provided a top layer of predominating hydrophobic properties and a bottom layer of predominating hydrophilic properties. The transfer layer has an embossed surface configuration, with channels formed by compressed nonwoven streaks forming transversal peaks and valleys extending in longitudinal direction along the transfer layer. The lower thickness of the transfer layer resulting from the compressed stretches which form valleys, provide the channels with a predominating hydrophilicity, which facilitates the quick liquid transfer towards the absorbent core, while the higher thickness of the transfer layer in the peaks provide it with a predominating hydrophobia, which impedes the liquid to return from the absorbent core to the permeable cover, thus reducing the index of remnant humidity in the zone of contact with the user's skin, even if the absorbent core is submitted to pressure.

Document EP 0 788 874 A1 discloses a gathered, layered composite material comprised of a fabric, film, foam or combination thereof that has a first layer of flexible reversibly elongatable material, bonded at spaced apart positions to at least one additional layer of a flexible material, wherein both layers are gathered, the gathers of one layer being aparallel to the gathers of the other layer. The composite material may be made by first reversibly tensioning, the first layer and concomitantly reversibly narrowing the first layer, in a direction coplanarly orthogonal to that in which tension is applied. The tensioned and narrowed first layer is then bonded discrete spaced apart positions to an at least one additional layer of a flexible nontensioned material, comprised of a fabric, film or foam or combination thereof. When these resulting tensioned and adhered layered materials are subsequently untensioned, they form a three dimensional layered composite material, wherein both layers are gathered, the gathers of one layer being aparallel to the gathers of the other layer. The layered composites of this invention may be used in the manufacture of various products including, but not limited to, sanitary absorbent products such as diapers, sanitary napkins, incontinence products, as well as bandages, wound dressings, surgical dressings, cushioning and bandages for the treatment of stasis ulcers, surgical drapes, underpads and the like.

Document US 4 578 070 A discloses an absorbent product having a first fibrous layer in the form of a nonwoven web and a second layer which is discrete from the first layer but united to the first layer. The second layer has a higher capillary pressure than the first layer to provide preferential attraction and wicking of liquid. The layers are corrugated and stabilized to retain the transverse folds when wet.

There is a need for improved acquisition and distribution layers providing improved flow, and more particularly improved directional flow, preferably down the length of the absorbent article to promote increased speed and amount of absorption and a distribution of fluids over the absorbent core which is suitable for and preferably adapted for the absorbent capacity of the absorbent core which can be location-dependent. Moreover, there is still a need for acquisition and distribution layers that more effectively prevent leakage, provide more comfort for the wearer, and reduce the surface wetness in the topsheet.

The present invention aims to resolve at least some of the problems mentioned above. The invention thereto aims to provide an improved acquisition distribution layer, also aims to provide an absorbent article which gives an improved feel for the wearer as well as a process for making such a layer and the use of such layered structure according to the invention in an absorbent article, particularly a diaper.

### SUMMARY OF THE INVENTION

The present invention concerns an absorbent article, preferably a disposable absorbent article, such as a diaper.

In a first aspect, the present invention specifically concerns an acquisition and distribution layer (6) suitable for an absorbent article, comprising a top layer (12) comprising staple fibers (22) and a, preferably essentially flat, bottom layer, the acquisition and distribution layer comprising an undulated structure. In a preferred embodiment, said top-layer comprises corrugations which at least partially define said undulated structure.

In a preferred embodiment, said undulated structure comprises at least two parallel tubes (20) defined by said corrugations and said bottom layer, at least two parallel channels (21) defined by said corrugations, and/or at least one tube (20) and at least one channel (22), parallel to said tube. More preferably, said tube is defined by two neighboring and essentially parallel channels and said bottom layer.

Fluids are substantially guided in a longitudinal direction through the channels (21) which can be further collected in the tubes (20) by e.g. capillary absorption that allows for fluids flow along the tubes (20). This promotes removal of a stationary portion of the fluids retained on the nonwoven topsheet (23) and results in a better distribution of the fluids through the acquisition and distribution layer (6), before penetrating an underneath absorbent core (18).

An acquisition and distribution layer (6) according to the present invention comprises a two-layered structure with differential permeability. Hereby, the top layer (12) comprises a higher permeability than the bottom layer (13), so that fluids can easily enter into the top layer (12), but are then slowed down rather than directly exiting through the bottom layer (13). This differential permeability enhances the liquid intake performance of the absorbent article and provides a temporary retention or absorption function for fluids not yet absorbed into the absorbent core (18), which tends to reduce leakage from the absorbent core (18) to the outside of the absorbent article. The as yet unabsorbed fluids thus can flow via the tubes to other areas of the ADL which can be located near areas of the absorbent core which are not yet saturated very much or have a larger absorbent capacity.

More in particular, insults typically occur in a relatively small area of 10 to 20 cm², whereby a large volume of fluid is discharged in a short time period, and this at irregular intervals. The present invention concerns an ADL which can be placed in an absorbent article between a topsheet and an absorbent core, and which is capable of delaying fluid from an insult from being absorbed by the absorbent core at the location of the insult, hereby allowing the fluid which is not absorbed immediately to be guided towards regions above the absorbent core which are less likely to be located near a previous, present or a future insult. Hereby, the absorbent core at the region of the insult is not immediately saturated at the first insult because a significant portion of the fluid from the insult is guided to other regions of the absorbent core to be absorbed there. Furthermore, the fluid which is not immediately absorbed is essentially guided in the undulated structure, e.g. the tubes or channels of the ADL and not via e.g. the topsheet of the absorbent article, which ensures a drier feeling to the wearer of the article. The ADL thus has three very important effects: (i) the absorbent capacity of the full absorbent core can be used; (ii) also at subsequent insults, at least a portion of the fluid can be absorbed immediately by the absorbent core at the location of the insult, as the absorbent capacity of this area of the absorbent core was not fully used for absorbing the first insult; and (iii) the undulated structure of the ADL, preferably the tubes and/or channels of the ADL, allow directional flow towards regions which are otherwise not accessed by fluids from insults, preferably the directional flow being longitudinal, i.e. in a front-back direction, with respect to an absorbent article in which the present ADL is used.

In a second aspect, the present invention provides an absorbent article comprising an acquisition and distribution layer (6) according to an embodiment of the invention wherein fluids are better distributed through the acquisition and distribution layer (6), before accessing the underneath bottom layer (13).

The present invention provides an absorbent article comprising an acquisition and distribution layer (6) wherein the undulated structure is oriented along a substantially longitudinal direction of the absorbent article, preferably the undulated structure comprises tubes and/or channels which are oriented along a direction which makes an angle of smaller than 45°, preferably smaller than 40°, more preferably smaller than 35°, yet more preferably smaller than 30°, still more preferably smaller than 25°, even more preferably smaller than 20°, yet even more preferably smaller than 15°, still even more preferably smaller than 10°, e.g. 9°, 8°, 7°, 6°, 5°, 4°, 3°, 2°, 1° or 0° with said longitudinal direction. Most preferably said channel structure comprises tubes and/or channels oriented about said longitudinal direction.

In a preferred embodiment, said undulated structure comprises only tubes and/or channels along a main direction, preferably within 25° from said main direction, more preferably within 20° from said main direction, yet more preferably within 15° from said main direction, still more preferably within 10° from said main direction, even more preferably within 5° from said main direction, e.g. within 4°, 3°, 2°, 1°, 0° from said main direction.

In a third aspect, the present invention provides a process for manufacturing an acquisition and distribution layer (6) according to claim 1.

In a further aspect, the invention also provides a process for manufacturing an absorbent article according to an embodiment of the invention, comprising the steps of providing an acquisition and distribution layer (6) according to the present invention to manufacture an absorbent article with an improved fluids distribution. The acquisition and distribution layer (6) improves the ability to prevent a feeling of wetness of the top layer (12). The undulated structure can redirect fluids from the channels (21) to the tubes (20) and can guide the fluid through the tubes, thereby limiting the contact with the skin of the diaper carrier.

In a final aspect, the invention provides for a use of an acquisition and distribution layer (6) according to an embodiment of the invention for manufacturing an absorbent article, in particular a diaper.

The acquisition and distribution layer (6) provided by the invention can be particularly advantageous for use in an absorbent article such as a diaper, preferably said absorbent article comprising an absorbent core, whereby the absorbent core is disposed against the bottom layer of said ADL.

Preferred embodiments are as specified below and in the claims.

### BRIEF DESCRIPTION OF THE DRAWINGS

The invention is further described in detail with reference to the exemplary embodiments represented by the accompanying figures, in which
**FIG. 1** shows a schematic cross-sectional view of an absorbent article according to the present invention;
**FIG. 2** shows a schematic cross-sectional view of an acquisition and distribution layer (6) belonging to the absorbent article from **FIG. 1** according to the present invention; and
**FIG. 3** shows a plan view of an embodiment of an absorbent article according to the present invention.
**FIG. 4** shows a plan view of an embodiment of another type of absorbent article according to the present invention.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

As used herein, the following terms have the following meanings:
"Absorbent article" refers to devices that absorb and contain liquid, and more specifically, refers to devices that are placed against or in proximity to the body of the wearer to absorb and contain the various exudates discharged from the body.
Absorbent articles include but are not limited to diapers, adult incontinence briefs, training pants, diaper holders and liners, sanitary napkins and the like.

"Diaper" refers to an absorbent article generally worn by infants and incontinent persons about the lower torso.

"Disposable" is used herein to describe articles that are generally not intended to be laundered or otherwise restored or reused (i. e., they are intended to be discarded after a single use and, preferably, to be recycled, composted or otherwise disposed of in an environmentally compatible manner).

"Nonwoven" refers to a manufactured sheet, web, or batt directionally or randomly oriented fibres bonded by friction and/or cohesion and/or adhesion, excluding paper and products that are woven, knitted, tufted stitchbonded incorporating binding yarns or filaments, or felted by wet-milling, whether or not additionally needled. The fibres may be of natural or man-made origin. They may also be staple or continuous filaments or be formed in situ.

An absorbent article of the present invention comprises a liquid permeable top sheet, a liquid impermeable back sheet, and an absorbent medium disposed between the top sheet and the back sheet. The article may also include one or more features such as, but not limited to, ears or side panels, leg cuffs, fastener components and/or a belt. The top sheet, back sheet and the absorbent medium could be made from any suitable material known to the person skilled in the art.

The term "top sheet" refers to a liquid permeable material sheet forming the inner cover of the absorbent article and which in use is placed in direct contact with the skin of the wearer. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. Further examples of top sheet materials are porous foams, apertured plastic films, laminates of nonwoven materials and apertured plastic films etc. The materials suited as top sheet materials should be soft and nonirritating to the skin and be readily penetrated by body fluid, e.g. urine or menstrual fluid. The inner coversheet may further be different in different parts of the absorbent article.

The "back sheet" refers to a material forming the outer cover of the absorbent article. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The "absorbent medium" is the absorbent structure disposed between the top sheet and the back sheet of the absorbent article in at least the crotch region thereof. The absorbent material can be of any conventional kind. Examples of commonly occurring absorbent materials are cellulosic fluff pulp, tissue layers, highly absorbent polymers (so called superabsorbents polymers (SAP)), absorbent foam materials, absorbent nonwoven materials or the like. It is common to combine cellulosic fluff pulp with superabsorbent polymers in an absorbent material. Superabsorbent polymers are water-swellable, water-insoluble organic or inorganic materials capable of absorbing at least about 20 times their own weight of an aqueous solution containing 0.9 weight percent of sodium chloride. Organic materials suitable for use as superabsorbent materials can include natural materials such as polysaccharides, polypeptides and the like, as well as synthetic materials such as synthetic hydrogel polymers. Such hydrogel polymers include, for example, alkali metal salts of polyacrylic acids, polyacrylamides, polyvinyl alcohol, polyacrylates, polyvinyl pyridines, and the like. Other suitable polymers include hydrolyzed acrylonitrile grafted starch, acrylic acid grafted starch, and isobutylene maleic anhydride copolymers and mixtures thereof. The hydrogel polymers are preferably lightly cross-linked to render the material substantially water insoluble. Preferred superabsorbent materials are further surface cross-linked so that the outer surface or shell of the superabsorbent particle, fibre, flake, sphere, etc. possesses a higher crosslink density than the inner portion of the superabsorbent. The superabsorbent materials may be in any form which is suitable for use in absorbent composites including particles, fibres, flakes, spheres, and the like.

The term "staple fibers" refers to commercially available fibers that comprise diameters ranging from less than about 0.001 mm to more than about 0.2 mm; they come in several different forms such as short fibers ranging from about 10 to 50 mm in length and long fibers with a length higher than 50 mm, preferably up to 100 mm. The staple fibers for use in the invention can be prepared from thin and/or coarse fibers, preferably polyester fibers. The thin fibers have a dtex below 3, preferably in the range of 1 to 3 whereas the heavy fibers have a dtex of at least 3 and preferably lighter than 45 dtex. In case a blend of thin and coarse fibers is used, the coarse fibres preferably have a dtex value which is at least two times, but no greater than 15 times that of the thin fibers.

The "hydroentanglement process" refers to the manufacturing of nonwoven webs. The process involves directing a series of water jets towards a fibrous web which is supported on a moving porous belt. The water jets pass downwards through the mass of fibres and on making contact with the surface of the belt, the jets rebound, and break up: the energy released causes entanglement of the mass of fibres.

The "acquisition and distribution layer" or "ADL", also referred to as sub-layer, is a, preferably nonwoven, wicking-type layer which is disposed under the top sheet (or face fabric) of an absorbent product, which speeds the transport and distribution of fluids throughout the absorbent core.

"Superabsorbent polymer particles" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc..., the particle size is preferably ranging between 100 to 800 µm, more preferably between 300 to 600 µm, yet more preferably between 400 to 500 µm.

Unless specified otherwise, the longitudinal dimension or length of an absorbent layer as used herein is to be understood as the average length.

Unless specified otherwise, the transverse dimension or width of an absorbent layer as used herein is to be understood as the average width.

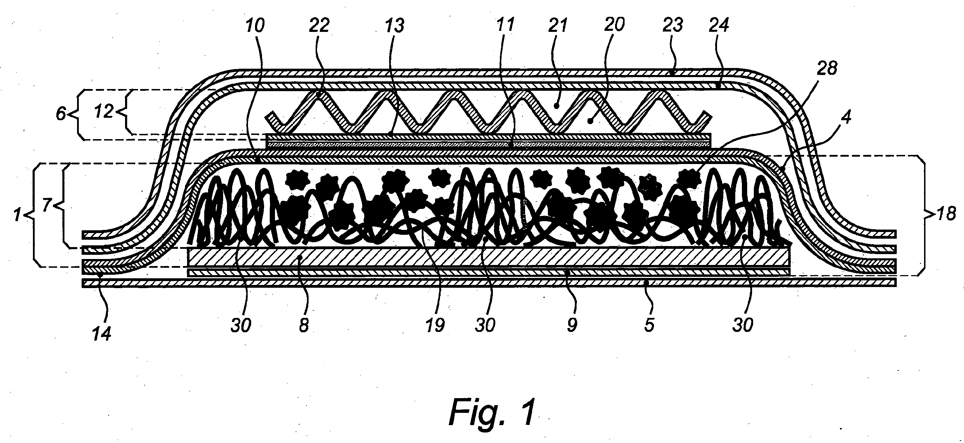

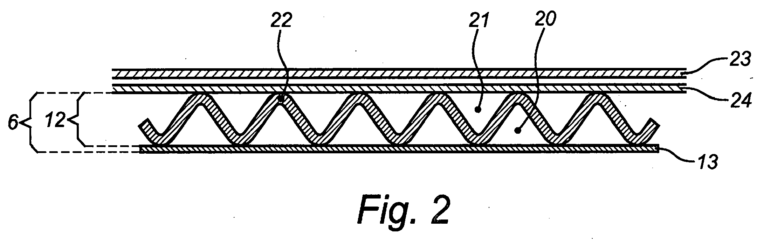

**Fig. 1** and **2** show two embodiments of an absorbent article according to the present invention. **Fig. 1** is a general schematic cross-sectional view of the absorbent article whereas **Fig. 2** is a detailed part of the schematic cross-sectional view of **Fig. 1** showing an acquisition and distribution layer (6).

The inventors have found a way to provide an improved acquisition distribution layer (6) and process for making such a layer. In particular, the present invention provides in a first aspect an acquisition and distribution layer (6) comprising a top layer (12) comprising staple fibers (22), and comprising an undulated structure, the structure preferably comprising a set of at least two essentially parallel tubes (20) and/or channels (21) and/or at least one tube (20) and at least one channel (21) essentially parallel to said tube.

In a preferred embodiment, the top layer comprises a blend of at least two types of fibers, preferably polyester fibers. Preferably, a first type of fibres is fine, more preferably thinner than 2.5 dtex, still more preferably thinner than 2.3 dtex, yet more preferably thinner than 2 dtex, yet even more preferably thinner than 1.5 dtex, most preferably about 1.3 dtex. Also preferable, a second type of fibres of said two types is coarse, more preferably thicker than 2.5 dtex, still more preferably thicker than 2.8 dtex, yet more preferably thicker than 3 dtex, yet even more preferably thicker than 3.2 dtex, most preferably about 3.3 dtex.

In a more preferred embodiment, the blend comprises at least 10%, more preferably at least 20%, yet more preferably at least 30%, still more preferably at least 40% of said first and/or said second type of fibres. Preferably, said blend comprises between 40% and 60% of said first type of fibers and between 40% and 60% of said second type of fibers, most preferably about 50% of the first type of fibres and 50% of the second type of fibers.

In a preferred embodiment, said top layer comprises fibres which comprise a length which is longer than 5 mm, preferably longer than 10 mm, more preferably longer than 15 mm, still more preferably longer than 20 mm, yet more preferably longer than 25 mm, even more preferably longer than 30 mm, still even more preferably longer than 35 mm, and/or said length shorter than 100 mm, preferably shorter than 90 mm, more preferably shorter than 80 mm, still more preferably shorter than 70 mm, yet more preferably shorter than 60 mm, even more preferably shorter than 50 mm, yet even more preferably shorter than 45 mm, still even more preferably shorter than 40 mm, e.g. 36 mm, 37 mm, 38 mm, 39 mm, most preferably about 38 mm.

Preferably, the fibers comprise a substantially round cross section, a substantially trilobal cross section and/or a substantially quadrilobal cross section.

In a preferred embodiment, the top layer comprises a grammage between 1 to 200 gsm, preferably 5 to 150 gsm, more preferably 10 to 100 gsm, most preferably 20 to 80 gsm, e.g. 30, 40, 50, 60, 70 gsm or any value therebetween, most preferably about 50 gsm.

In a preferred embodiment, the bottom layer (13) comprises a spunbond-meltblown-spunbond polypropylene nonwoven.

In a preferred embodiment, the bottom layer comprises a grammage between 0,5 to 50 gsm, preferably 1 to 40 gsm, more preferably 2 to 30 gsm, more preferably 5 to 20 gsm, most preferably 10 to 15 gsm, e.g. 11, 12, 13, 14 gsm or any value there between, most preferably about 13 gsm.

In a preferred embodiment, the ADL is hydrophilic, more preferably said top layer and said bottom layer are hydrophilic.

In a preferred embodiment, said ADL, said top layer and/or said bottom layer are treated with a surfactant, preferably with a surfactant rendering said ADL, said top layer and/or said bottom layer hydrophilic.

In a preferred embodiment, the ADL comprises a maximum length of between 5 cm and 50 cm, more preferably between 10 cm and 40 cm, yet more preferably between 20 cm and 30 cm, and a maximum width of between 2.5 cm and 30 cm, more preferably between 5 cm and 20 cm, still more preferably between 7.5 cm and 15 cm.

In a preferred embodiment, the undulated structure comprises two neighboring tubes (20) separated by a channel (21) and/or two neighboring channels (21) separated by a tube, whereby said two neighboring tubes and/or channels comprise a separating distance of between 3 mm and 20 mm, preferably between 5 mm and 15 mm, more preferably between 8 mm and 13 mm, still more preferably 9, 10, 11 or 12 mm, the distance being measured between the center of the neighboring tubes or channels (21), to improve fluids distribution, and a nonwoven bottom layer (13) (**Fig. 1**).

The invention is characterised in that the acquisition and distribution layer (6) is based upon Nonwovens Innovation & Research Institute's proprietary Hydrospace technology, as described in EP 1644564A1. Therefore, the ADL according to the present invention comprises a thin, 3D nonwoven spacer fabric with discrete, channel-like voids or cells within the fabric cross-section, which relies on fluid forces rather than conventional mechanical methods to periodically interconnect, e.g. hydro-entagle, fibres from at least two web structures which are separated by a spacer system during their production. The ADL comprises a nonwoven fabric, the fabric comprising at least two separate but interconnected layers, each of the layers being provided with discrete interconnections so as to provide discrete voids between the two layers of fabric. The shape of the voids may vary. However, preferentially, the voids comprise a channel and/or a tube, e. g. a plurality of channels and/or tubes within the structure of the fabric. The channels and/or tubes may be substantially cylindrical in shape. However, it will be understood by one skilled in the art that the size and/or shape of the voids may be influenced by the choice of the spacer material. Similarly, the size of the voids may vary, depending, inter alia, on the nature of the use of the nonwoven fabric. However, preferentially, the channels and/or tubes are such that they comprise a diameter in the range of from. 0.2 mm to 8.5 mm, more preferably from 0.5 mm to 6 mm, yet more preferably from 1 mm to 5 mm, e.g. 2 mm, 3 mm, 4 mm or any value therebetween.

In a preferred embodiment, the undulated structure comprises between 3 and 30 tubes, more preferably between 5 and 25 tubes, still more preferably between 7 and 20 tubes, e.g. 8, 9, 10, 11, 12, 13, 14, 15, 16, 17, 18 or 19 tubes, which are oriented along a main direction, preferably within 25° from said main direction, more preferably within 20° from said main direction, yet more preferably within 15° from said main direction, still more preferably within 10° from said main direction, even more preferably within 5° from said main direction, e.g. within 4°, 3°, 2°, 1°, 0° from said main direction.

In a preferred embodiment, the undulated structure comprises a tube-density along a direction perpendicular to the main direction and essentially parallel to the ADL, in between 0.2 and 5 tubes per cm, more preferably between 0.4 and 3 tubes per cm, still more preferably between 0.6 and 2 tubes per cm, e.g. 0.7, 0.8, 0.9, 1, 1.1, 1.2, 1.3, 1.4, 1.5, 1.6, 1.7, 1.8, 1.9 tubes per cm.

The acquisition and distribution layer (6) can preferably be attached by thermoplastic adhesive (11) to an underneath absorbent core (18). The absorbent core (18) and the acquisition and distribution layer (6) being covered by a nonwoven topsheet (23) attached to the components underneath with a layer of thermoplastic adhesive (24). This is particularly preferred for absorbent articles comprising such an ADL or if such an ADL is to be used in an absorbent article. Note however, that any type of adhesive application knwon in the art could be used to attach the ADL of the present invention to other components or layers of an absorbent article. In particular, adhesion can be obtained by gluing, such by contact coating with full coverage or partial coverage, e.g. in stripes, or by spray coating, e.g. random spray coating or along a pattern, continuous or discontinuous, e.g. by non-continuous lines of spiral spray. Adhesion can also be obtained by alternative bonding techniques, such as thermal bonding, thermo-mechanical bonding, mechanical bonding and/or ultrasonic bonding.

Fluids are substantially guided in the longitudinal direction through the channels (21) which are further collected in the tubes (20) by e.g. capillary absorption that allows for the fluids to flow along the tubes (20). This promotes removal of a stationary portion of the fluids present on the nonwoven topsheet (23) and results in a better distribution of the fluids through the acquisition and distribution layer (6), before penetrating an underneath absorbent core (18).

The absorbent core (18) can preferably be wrapped between two layers of nonwoven, the so-called core wrap. The lower core wrap (5) can be attached to the bottom layer (8) of a nonwoven carrier (1) by thermoplastic adhesive (9). The upper core wrap (4) can be attached to the top surface of the nonwoven carrier (1) by thermoplastic adhesive (10). Superabsorbent polymer particles (28) can preferably be partly in contact with the adhesive coating (10) on the upper core wrap (4). The superabsorbent particles (SAPs) are preferably distributed according to a pattern, which preferably comprises areas (30) which are essentially free from SAPs, such as is the case in a clustered pattern. Preferably, the widths of the core wrap nonwovens exceed the width of the nonwoven carrier (1), so that the core wrap nonwovens can be bonded with each other along the side edges by thermoplastic adhesive (14).

The acquisition and distribution layer (6) according to the present invention comprises an at least two-layered structure with differential permeability. The top layer (12) has a higher permeability than the nonwoven bottom layer (13), so that fluids can easily enter into the top layer (12), but are then slowed down rather than directly exiting through the nonwoven bottom layer (13). This differential permeability enhances the liquid intake performance of the absorbent article and provides a temporary retention or absorption function for fluids not yet absorbed into the absorbent core (18), which tends to reduce leakage from the absorbent core (18) to the outside of the absorbent article.

The differential permeability allows to transport fluids in a fractionated way, first through the top layer (12) and then temporarily retained through the nonwoven bottom layer (13). The acquisition and distribution layer (6) serves as a temporary reservoir to collect and hold fluids until the fluids can be absorbed by the core (18). One of the advantage is that fluids are guided in a structured way, lowering the risk of leakage.
In a preferred embodiment, the acquisition and distribution layer (6) is configured in an undulated structure wherein the tubes (20) comprise a diameter of 2 mm.

In a preferred embodiment, the tube (20) comprise a diameter of at least 0.1 mm, preferably at least 0.2 mm, more preferably at least 0.5 mm, even more preferably at least 0.8 mm, and/or preferably at most 10 mm, more preferably at most 8 mm, more preferably at most 5 mm, even more preferably at most 3 mm, e.g. 0.8; 1; 1.5; 2; 2.5; 3 mm, most preferably about 1.07 mm. The tubes (20) are substantially cylindrical in shape. The size of the tubes (20) may vary, depending on the nature of the use of the acquisition and distribution layer (6). However, preferentially, the tubes (20) comprise a diameter of about 1.07 mm. The arrangement of the tubes (20) may be ordered or disordered. However, it is preferred that the tubes (20) are arranged in a substantially uniform manner.

In a preferred embodiment, the nonwoven bottom layer (13) is substantially flat. The flat structure allows to collect fluids coming from the undulated structure in a homogeneous way. Indeed, fluids will spread over the surface of the nonwoven bottom layer (13) resulting in a more homogeneous distribution of the fluids over the surface. This will lead to a better control of the fluids flow on the nonwoven bottom layer (13) before accessing an underneath absorbent core (18), thereby avoiding unpleasant leakage for the diaper wearer.

In a preferred embodiment, the staple fibers (22) are covered by a nonwoven topsheet (23) which is attached by a layer of thermoplastic adhesive (24).

In a second aspect, the invention provides an absorbent article comprising an acquisition and distribution layer (6) according to an embodiment of the invention.

In a preferred embodiment, the invention provides an absorbent article comprising an acquisition and distribution layer (6) wherein the undulated structure is oriented along a main direction of the acquisition and distribution layer (6) which makes an angle of smaller than 45°, preferably smaller than 40°, more preferably smaller than 35°, yet more preferably smaller than 30°, still more preferably smaller than 25°, even more preferably smaller than 20°, yet even more preferably smaller than 15°, still even more preferably smaller than 10°, e.g. 9°, 8°, 7°, 6°, 5°, 4°, 3°, 2°, 1° or 0° with a longitudinal axis of said absorbent article.

In a third aspect, the present invention provides a process for manufacturing an acquisition and distribution layer (6) according to an embodiment of the invention, comprising the steps of interconnecting staple fibers (22) and nonwoven bottom layer (13) forming an undulated structure, preferably comprising a tube and/or a channel comprising a substantially tubular shape. The tubular shape of the tube or channel is formed by hydroentangling the top layer (12) to the nonwoven bottom layer (13). Preferably, the tube and/or channel comprise a maximum length of between 5 cm and 50 cm, more preferably between 10 cm and 40 cm, yet more preferably between 20 cm and 30 cm, and a maximum width of between 2.5 cm and 30 cm, more preferably between 5 cm and 20 cm, still more preferably between 7.5 cm and 15 cm.

The staple fibers (22) and the nonwoven bottom layer (13) are bonded by hydroentanglement, e.g. as according to EP 1644564A1.

The basic technology of high pressure water jets used to produce hydroentangled materials, is employed as the method of manufacturing 3D nonwoven spacer fabrics. It is intended that at least two fibrous layers will be separated using different spacer devices of various geometrical designs. Whilst in contact with the spacer device, these layers will be impacted by water jets to both entangle fibres in the individual layers and to migrate groups of fibres in to the Z-direction to interconnect the adjacent layers between spacer bar elements. In this way, the microstructure of internal channel-like voids/pores in the fabric is influenced by the shape and morphology of the spacer device used (which may be repeated across the width of the fabric), process conditions and fiber properties. In a simple embodiment, the spacer device may be an array of smooth, hollow cylindrical rods of different sizes or cross-sections arranged across the width of the machine. Many different designs are possible. Also, secondary bonding of the structure using such means as thermal or chemical bonding can be utilised to stabilise the internal void structures produced during the process. This improves the dimensional stability of the internal channel-like void structures and consequently the physical properties of the fabric will be enhanced as required for specific applications.

In a preferred embodiment, the process further comprises the step of treating the top layer and/or the bottom layer, and preferably the ADL, with a surfactant after the hydroentanglement process. The top layer, bottom layer and/or ADL is rendered hydrophilic through the use of a post treatment application of a surfactant. The hydrophilic characteristic of the top layer, bottom layer and/or ADL improves absorbent articles such as baby diaper and adult incontinence diaper by improving absorbency.

In a further aspect the invention provides a process for manufacturing an absorbent article according to an embodiment of the invention, which comprises the use of an acquisition and distribution layer (6) according to the present invention.

In a preferred embodiment, the acquisition and distribution layer (6) improves the ability to prevent a feeling of wetness of the top layer (12). The undulated (20,21) structure redirects fluids from the channels (21) to the tubes (20) thereby limiting the contact with the skin of the diaper wearer.

In a final aspect, the invention provides for a use of an acquisition and distribution layer (6) according to an embodiment of the invention for manufacturing an absorbent article, in particular a diaper.

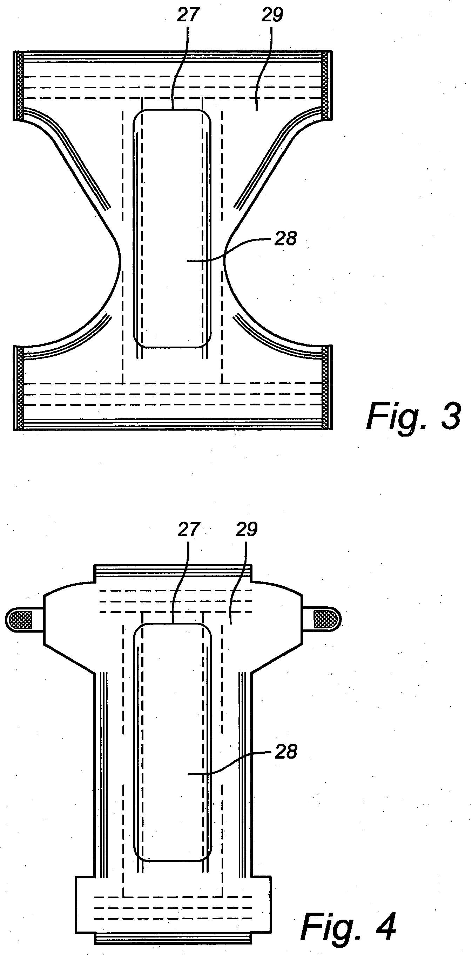

**FIGS. 3 and 4** are plan views of disposable diapers of a pant-type (adult) incontinence diaper and a baby diaper according to a certain embodiment of the present invention. The disposable diaper is shown in its flat out, uncontracted state (i.e. without elastic induced contraction). The central part of the disposable diaper (27) is shown which is limited by the outer part of the disposable diaper (29).

Although the present invention has been described with reference to preferred embodiments thereof, many modifications and alterations may be made by a person having ordinary skill in the art without departing from the scope of this invention which is defined by the appended claims.

## Claims

1. Method for manufacturing an acquisition and distribution layer (6) (ADL), comprising the steps of interconnecting a top layer comprising staple fibers (22) and a bottom layer (13) by hydro-entanglement, thereby forming an undulated structure, whereby the top layer (12) comprises a higher permeability than the bottom layer (13), **characterized in that** said undulated structure is formed by hydro-entangling fibers from at least two web structures which are separated by a spacer system during their production.

2. Method according to claim 1, comprising the step of treating the ADL with a surfactant rendering said ADL hydrophilic.

3. An acquisition and distribution layer (6) (ADL) suitable for an absorbent article and obtainable by a method according to any of the previous claims, comprising a top layer (12) comprising staple fibers (22) and a bottom layer, the acquisition and distribution layer comprising an undulated structure, whereby the top layer (12) comprises a higher permeability than the bottom layer (13), **characterized in that** the top layer is hydro-entangled to the bottom layer at discrete interconnections , whereby discrete voids are provided between the two layers of fabric, said voids comprising a channel and/or a tube.

4. The ADL according to claim 3, whereby the bottom layer is substantially flat and said top-layer comprises corrugations which at least partially define said undulated structure.

5. The ADL according to any of the claims 3 to 4, whereby said ADL, said top layer and/or said bottom layer are hydrophilic.

6. The ADL according to any of the claims 3 to 5, where said bottom layer comprises a spunbond-meltblown-spunbond nonwoven.

7. The ADL according to any of the claims 3 to 6, comprising:
- a maximum length of between 5 cm and 50 cm;
- a maximum width of between 2.5 cm and 30 cm; and
- at least two neighboring tubes (20) separated by a channel (21) and/or two neighboring channels (21) separated by a tube, whereby said two neighboring tubes and/or channels comprise a separating distance of between 3 mm and 20 mm, the distance being measured between the center of said neighboring tubes or channels (21).

8. The ADL according to any of the claims 3 to 7, whereby the undulated structure comprises between 3 and 30 tubes, which are oriented along a main direction within 5° from said main direction.

9. The ADL according to any of the claims 3 to 8, wherein the staple fibers are covered by a nonwoven topsheet which is attached to the staple fibers by a layer of adhesive.

10. Absorbent article comprising an acquisition and distribution layer (ADL) manufactured by a method according to any of the claims 1 to 2 or an ADL according to any of the claims 3 to 9.

11. Absorbent article according to claim 10, wherein the undulated structure of the ADL is oriented along a longitudinal direction of the absorbent article.

12. Absorbent article according to any of the claims 10 to 11, comprising an absorbent core disposed underneath the bottom layer of the ADL.

13. Use of an acquisition and distribution layer manufactured by a method according to any of claims 1 to 2 or an ADL according to any of the claims 3 to 9, for manufacturing an absorbent article.

## Patentansprüche

1. Verfahren zur Herstellung einer Aufnahme- und Verteilungsschicht (6) (ADL), umfassend die Schritte vom Verbinden einer oberen Schicht umfassend Stapelfasern (22) und einer unteren Schicht (13) durch Wasserstrahlverwirbelung, wodurch eine gewellte Struktur gebildet wird, wobei die obere Schicht (12) eine höhere Durchlässigkeit als die untere Schicht (13) umfasst, **dadurch gekennzeichnet, dass** die gewellte Struktur gebildet wird durch das Wasserstrahlverwirbeln von Fasern aus mindestens zwei Vliesstrukturen, die durch ein Abstandhaltersystem während ihrer Herstellung getrennt sind.

2. Verfahren nach Anspruch 1, umfassend den Schritt des Behandelns der ADL mit einem Tensid, was die ADL hydrophil macht.

3. Eine Aufnahme- und Verteilungsschicht (6) (ADL) geeignet für einen absorbierenden Artikel und erhältlich durch ein Verfahren nach einem der vorhergehenden Ansprüche, umfassend eine obere Schicht (12) umfassend Stapelfasern (22) und eine untere Schicht, die Aufnahme- und Verteilungsschicht umfassend eine gewellte Struktur, wobei die obere Schicht (12) eine höhere Durchlässigkeit als die untere Schicht (13) umfasst, **dadurch gekennzeichnet, dass** die obere Schicht an diskreten Zwischenverbindungen zu der unteren Schicht wasserverwirbelt ist, wodurch diskrete Hohlräume zwischen den beiden Stoffschichten vorgesehen sind, die Hohlräume umfassend einen Kanal und/oder ein Rohr.

4. Die ADL nach Anspruch 3, wobei die untere Schicht im Wesentlichen flach ist und die obere Schicht Wellungen umfasst, die zumindest teilweise die gewellte Struktur definieren.

5. Die ADL nach einem der Ansprüche 3 bis 4, wobei die ADL, die obere Schicht und/oder die untere Schicht hydrophil sind.

6. Die ADL nach einem der Ansprüche 3 bis 5, wobei die untere Schicht ein spinngebundenen-schmelzgeblasenen- spinngebundenen Vliesstoff umfasst.

7. Die ADL nach einem der Ansprüche 3 bis 6, umfassend:
- eine maximale Länge zwischen 5 cm und 50 cm;
- eine maximale Breite zwischen 2,5 cm und 30 cm; und
- mindestens zwei benachbarte Rohre (20) durch einen Kanal (21) getrennt und/oder zwei benachbarte Kanäle (21) durch ein Rohr getrennt, wobei die zwei benachbarten Rohre und/oder Kanäle einen Trennabstand zwischen 3 mm und 20 mm umfassen, wobei der Abstand gemessen wird zwischen der Mitte der benachbarten Rohre oder Kanäle (21).

8. Die ADL nach einem der Ansprüche 3 bis 7, wobei die gewellte Struktur zwischen 3 und 30 Rohre umfasst, die entlang einer Hauptrichtung innerhalb von 5° von der Hauptrichtung orientiert sind.

9. Die ADL nach einem der Ansprüche 3 bis 8, wobei die Stapelfasern von einer Vliesstoffdecklage, die zu den Stapelfasern durch eine Klebstoffschicht befestigt ist, bedeckt sind.

10. Absorbierender Artikel umfassend eine Aufnahme- und Verteilungsschicht (ADL) durch ein Verfahren nach einem der Ansprüche 1 bis 2 hergestellt oder eine ADL nach einem der Ansprüche 3 bis 9.

11. Absorbierender Artikel nach Anspruch 10, wobei die gewellte Struktur der ADL entlang einer Längsrichtung des absorbierenden Artikels orientiert ist.

12. Absorbierender Artikel nach einem der Ansprüche 10 bis 11, umfassend einen absorbierenden Kern, der unterhalb der unteren Schicht der ADL angeordnet ist.

13. Verwendung einer Aufnahme- und Verteilungsschicht durch ein Verfahren nach einem der Ansprüche 1 bis 2 hergestellt oder einer ADL nach einem der Ansprüche 3 bis 9, zur Herstellung eines absorbierenden Artikels.

## Revendications

1. Procédé de fabrication d'une couche d'acquisition et de distribution (6) (ADL), comprenant les étapes d'interconnecter une couche supérieure comprenant des fibres discontinues (22) et une couche inférieure (13) par l'hydro-enchevêtrement, ainsi former une structure ondulée, dans lequel la couche supérieure (12) comprend une perméabilité supérieure à la couche inférieure (13), **caractérisé en ce que** ladite structure ondulée est formée par hydro-enchevêtrer des fibres à partir d'au moins deux structures de nappe qui sont séparées par un système d'espacement pendant leur production.

2. Procédé selon la revendication 1, comprenant l'étape de traiter l'ADL avec un agent tensioactif, rendant l'ADL hydrophile.

3. Une couche d'acquisition et de distribution (6) (ADL) convenable pour un article absorbant et pouvant être obtenue par un procédé selon l'une quelconque des revendications précédentes, comprenant une couche supérieure (12) comprenant des fibres discontinues (22) et une couche inférieure, la couche d'acquisition et de distribution comprenant une structure ondulée, dans laquelle la couche supérieure (12) comprend une perméabilité supérieure à la couche inférieure (13), **caractérisée en ce que** la couche supérieure est hydro-enchevêtrée à la couche inférieure aux interconnexions discrètes, dans laquelle des vides discrets sont prévus entre les deux couches de tissu, lesdits vides comprenant un canal et/ou un tube.

4. L'ADL selon la revendication 3, dans laquelle la couche inférieure est substantiellement plate et ladite couche supérieure comprend des ondulations qui au moins partiellement définissent ladite structure ondulée.

5. L'ADL selon l'une des revendications 3 à 4, dans laquelle ladite ADL, ladite couche supérieure et/ou ladite couche inférieure sont hydrophiles.

6. L'ADL selon l'une quelconque des revendications 3 à 5, dans laquelle ladite couche inférieure comprend un non-tissé spunbond-meltbown-spunbond.

7. L'ADL selon l'une quelconque des revendications 3 à 6, comprenant:
- une longueur maximale comprise entre 5 cm et 50 cm;
- une largeur maximale comprise entre 2,5 cm et 30 cm; et
- au moins deux tubes voisins (20) séparés par un canal (21) et/ou les deux canaux voisins (21) séparés par un tube, dans laquelle lesdits deux tubes et/ou canaux voisins comprennent une distance de séparation comprise entre 3 mm et 20 mm, la distance étant mesurée entre le centre desdits tubes ou canaux voisins (21).

8. L'ADL selon l'une quelconque des revendications 3 à 7, dans laquelle la structure ondulée comprend entre 3 et 30 tubes, qui sont orientés le long d'une direction principale à moins de 5° de ladite direction principale.

9. L'ADL selon l'une quelconque des revendications 3 à 8, dans laquelle les fibres discontinues sont couvertes par une feuille de non-tissé qui est fixée aux fibres discontinues par une couche d'adhésif.

10. Article absorbant comprenant une couche d'acquisition et de distribution (ADL) fabriquée par un procédé selon l'une quelconque des revendications 1 à 2 ou une ADL selon l'une quelconque des revendications 3 à 9.

11. Article absorbant selon la revendication 10, dans laquelle la structure ondulée de l'ADL est orientée le long d'une direction longitudinale de l'article absorbant.

12. Article absorbant selon l'une quelconque des revendications 10 à 11, comprenant un noyau absorbant disposé au-dessous de la couche inférieure de l'ADL.

13. L'utilisation d'une couche d'acquisition et de distribution fabriquée par un procédé selon l'une quelconque des revendications 1 à 2 ou une ADL selon l'une quelconque des revendications 3 à 9, pour la fabrication d'un article absorbant.
